# EUROPEAN PATENT APPLICATION

(11) **EP 4 101 381 A1**
(43) Date of publication of application: **14.12.2022**
(21) Application number: 22175388.2
(22) Date of filing: 25.05.2022
(51) Int. Cl.: A61B 5/245, A61B 5/00

(54) **MAGNETOENCEPHALOGRAPHY APPARATUS**

(30) Priority: 11.06.2021 GB 202108360
(71) Applicant: The University of Nottingham, Nottingham, Nottinghamshire NG7 2RD (GB)
(72) Inventor: Brookes, Matthew, Nottingham, NG7 2RD (GB); Boto Barriga, Elena, Nottingham, NG7 2RD (GB); Bowtell, Richard, Nottingham, NG7 2RD (GB); Sims, Dominic, Nottingham, NG7 2RD (GB); Holmes, Niall, Nottingham, NG7 2RD (GB); Hill, Ryan, Nottingham, NG7 2RD (GB)
(74) Representative: Brickell, Stephanie Louise

(57) **Abstract**

A magnetoencephalography (MEG) apparatus comprising a helmet shaped and configured to fit a range of human head sizes and/or shapes, the helmet comprising a plurality of openings disposed in predetermined locations around the helmet, each opening being adapted to receive a magnetoencephalography field sensor in an arrangement such that the magnetoencephalography field sensor is moveable in a direction towards or away from a human head inside the helmet

## Description

### FIELD OF THE INVENTION

The present invention relates to apparatus for use in magnetoencephalography.

### BACKGROUND ART

Magnetoencephalography (usually known as MEG) is a medical imaging technique which measures the magnetic fields generated by the brain non-invasively using arrays of magnetic field sensors that are placed on or held near to the scalp. MEG presents a significant engineering challenge as the magnetic fields from the brain (about 10 femtotesla (fT)) are more than a hundred billion times smaller than the Earth's magnetic field (~50 microtesla (µT)), and many orders of magnitude smaller than other sources of magnetic interference, such as the fields generated by electronic devices, cars, lifts, mains electricity, etc (on the order of 10⁸ fT or 0.1 µT). The human brain can be thought of as a highly complex electrical circuit, containing hundreds of billions of current-carrying neurons. Just like electric currents flowing through wires, the tiny currents that flow through neurons generate magnetic fields. By measuring these magnetic fields it is possible to obtain a unique insight into brain function with millimetre precision, which is critical to identifying clinical markers such as the site of epileptic activity, and millisecond temporal resolution, which is key to studying real-time changes in brain state as it responds to the ambient environment. Typically, MEG systems will be employed within magnetically shielded spaces so that normal environmental magnetic noise is blocked so that the magnetic fields generated by the brain can be detected and measured.

For MEG to be accurate, the array of magnetic field sensors should be placed as close as possible to the scalp to be as close as possible to the source of the magnetic field.

Until recently, all MEG systems have used Superconducting QUantum Interference Devices (SQUIDs) for measuring the tiny magnetic fields from the brain. SQUIDs are among the most sensitive magnetic field detectors available but require cryogenic cooling with liquid helium which is at -269 °C. All SQUID-based MEG systems feature a fixed array of sensors inside a bath of liquid helium. The subject places their head inside a 'helmet' and is instructed to remain still; only movements less than 5 mm over a 20-minute period can be tolerated, thus preventing studies on patients with movement disorders such as Parkinson's disease. The SQUIDs are located far from the scalp due to a thermally insulating vacuum between the participant and the liquid helium dewar. For an adult this distance is 2-5 cm, and even further for infants and children which prevents the accurate MEG scanning of such a subject group. The costs and restrictions associated with MEG (the necessary one-size-fits-all design prohibits the scanning of participants with small heads (e.g. infants), and those who struggle to remain still during scans, such as patients with Parkinson's disease or Tourette's syndrome) have limited the application of MEG techniques.

A relatively recent type of magnetic field sensor, known as the Optically Pumped Magnetometer (OPM), has seen a revolution in MEG technology. OPMs have enabled the development of wearable systems which can be adapted to many different scanning situations. For example, so-called OPM-MEG systems have been used to scan adult participants whilst they are moving, children as young as 2 years' old, and patients with Tourette's syndrome, all of which would be impossible with a conventional system. An exciting feature of OPM-MEG is the ability to place OPMs anywhere, meaning bespoke helmets can be made which allow the sensors to move with the head. This enables exciting new experiments, but finding the positions and orientations of the sensors in these flexible systems presents new challenges.

In order to ensure the necessary accuracy in sensor location and orientation relative to the participant's skull, 3D-printed helmets based on an accurate MRI image of the subject being scanned have been used. Sensor holders are incorporated into the helmet structure so that, for this particular participant, OPMs can be placed directly onto the scalp with exact knowledge of where the sensors are and which direction they point in. However, the use of subject-specific helmets is expensive, the time to manufacture each helmet is extensive, the helmets need to be stored between patient visits and the correct helmet retrieved for each patient, and it is a lengthy process to switch sensors from helmet to helmet for different patients.

### SUMMARY OF THE INVENTION

The present invention provides magnetoencephalography (MEG) apparatus comprising a helmet shaped and configured to fit a range of human head sizes and/or shapes, the helmet having a fixture for fixing the helmet securely in position on a human head, a plurality of openings disposed in predetermined locations around the helmet, each opening being adapted to receive a magnetoencephalography field sensor in an arrangement such that the magnetoencephalography field sensor is moveable in a direction towards or away from a human head inside the helmet.

Such an arrangement allows a single, generic helmet to be used by different participants with different sized and shaped heads, whilst at the same time allowing the sensors to be positioned in contact with and relative to the participant's scalp with the required positional and orientational accuracy. Because the openings are preferably dispersed at predetermined locations around the helmet, and the openings are aligned in a known direction towards the participant's scalp, the sensors can be adjusted so as to protrude relatively more or less from a known datum surface (either the internal surface of the helmet, or an idealised or theoretical surface) inside the helmet and thus accommodate different head shapes and sizes. Thus the arrangement allows a single adult helmet to accommodate up to 95% of adult head sizes, which reduces considerably the amount of storage required for helmets needed for those outside this range (e.g. children, or those with very large or unevenly shaped heads); this therefore represents a significant cost saving over conventional systems. Moreover, the adaptable protrusion of the sensors may be used to distribute the weight of the helmet more evenly over the patient's head, making it more comfortable to wear than conventional helmets. Overall, this design combines the flexibility of the generic system, with the benefits of a subject-specific helmet ensuring that the collected OPM-MEG data is of the highest possible quality.

The helmet may be sized according to data reflecting an average head size of an adult human subject. The helmet may also be provided in a range of standardised sizes (small, medium and large, for example) to better accommodate variations in head size based on subject gender, age range, and/or any medical condition affecting the size and shape of the human skull.

The magnetoencephalography field sensor is preferably an Optically Pumped Magnetometer (OPM), although this invention may be equally advantageously applicable with other MEG sensors. Preferably there is a continuous mechanism which allows the movement of the sensor relative to be infinitely variable (rather than being moveable between several fixed positions). Because magnetic fields decay with an inverse square law, the closer the sensors are to the source of the magnetic fields (i.e. the brain) the stronger the signals will be, but also the spatial variation of the field will be more complex. Combining this increase in signal with richer spatial information enables a more accurate determination as to where the neurons responsible for generating the measured fields are. It is also necessary to know the exact position and orientation of the sensors with respect to the head. If accurate locations of the sensors are not known, nor are the directions the sensors were pointing, then it is not possible to trace the measured magnetic fields back to the neurons that originally generated those fields. For the required millimetre precision images of brain activity, it is necessary to have sub-millimetre positional information and sub-degree orientation information of the magnetic field sensors with respect to the brain.

There may be at least two magnetoencephalography field sensors, each received in an opening in the helmet, at least one of the magnetoencephalography field sensors and/or the opening in which it is received being provided with indicia showing the position of the magnetoencephalography field sensor relative to the opening in the direction towards or away from a human head inside the helmet. This would allow the sensors to be mounted into the helmet openings, and then moved inwards towards the participant's head or outwards away from the participant's head so as to provide the most comfortable fit. The indicia can then be easily read, so that the locations of the sensing ends of the sensors can be determined in relation to the helmet, and thus in three-dimensional space, thus giving the necessary information to interpret the information signals received by each sensor with the required degree of accuracy to be able to monitor brain function within the brain with millimetric precision or better.

The or each magnetoencephalography field sensor may be adapted to move in a spiral path relative to helmet to move in the direction towards or away from a human head inside the helmet. This can be achieved with known mechanisms which convert rotational motion into axial motion, such as the mechanism used in lipstick devices; such mechanisms are simple, robust, and can be manufactured to give the required degree of accuracy. Also, where indicia indicating the sensor position are used with such a mechanism, these can be in the form of an easily read circular or part-circular dial. The mechanism may be provided with mechanical "stops", means which give a haptic signal as the sensor is rotated between predetermined rotational positions (which of course relate directly to specific axial positions, or positions along the direction towards/away from the participant's head).

The or each magnetoencephalography field sensor may conveniently be mounted in a housing which is releasably mountable within the openings in the helmet, with the sensor being movable with relation to the housing so as to provide the capability of movement of the magnetoencephalography field sensor in a direction towards or away from a human head inside the helmet when the housing is mounted to an opening. This allows the sensor and its housing to be manufactured as one assembly, so that the sensor is selectively movable along an axis in two directions relative to the housing; when the housing is inserted (preferably "snap-fitted") into an opening, whose position and orientation relative to the helmet is known, the orientation and direction of movement of the sensor relative to the helmet is easily calculated. This arrangement allows a single sensor housing to be used in helmets of any shape or size, and for the openings in the helmet to be spaced and oriented entirely as desired. It will be understood also that the helmet may comprise many openings, but that it may not be necessary to insert a sensor into all of the openings, it may only be necessary to use some of the openings (for example where only a specific region of the brain is to be studied); this further increases the flexibility of apparatus in accordance with the invention. Additionally or alternatively, some openings in the helmet may have dummy housings mounted in them - that is, housings which extend and retract in the same manner as sensor-equipped housings so as to help locate the helmet comfortably on a patient's head, but without any sensor being fitted.

It will also be appreciated that at least one of the magnetoencephalography field sensors and/or the housing(s) in which it is/they are mounted may be provided with indicia showing the position of the magnetoencephalography field sensor relative to the housing in the direction towards or away from a human head inside the helmet. In this way, the manufacture of the housings, with the one-dimensional movement of the sensor, and the application of the indicia indicating the axial position of the sensing end of the sensor, can be kept separate from the manufacture of the three-dimensional helmet.

The helmet may be provided with apertures between the openings, and/or it may have an open lattice structure surrounding the openings. This allows the helmet to be lighter, and thus less uncomfortable to wear, and also permits airflow to circulate around the participant's head and the sensors, thus removing excess heat away from the participant's head, and again aiding subject comfort (OPM sensors can reach temperatures of around 40° C which, although not hot enough to be painful, would be uncomfortable for a person to endure for any prolonged period). The better airflow which can be provided to remove excess heat, the less a participant is likely to perspire, and therefore the present invention is also intrinsically more hygienic than conventional arrangements, and requires less intensive cleaning. The apertures or openings also reduce the weight of the helmet, which aids the relaxation of the wearer and increases comfort.

In a preferred embodiment, the helmet may be rigid so that the accuracy of positioning of the of the or each opening, and by extension the or each magnetic field sensor, may be recorded. The helmet may be fabricated of suitable material to provide a desired level of rigidity and suitable mechanical characteristics of material that permit the formation of openings and/or a lattice structure present to allow for cooling and weight reduction.

In an alternative embodiment, the material of the helmet may be selected to provide a degree of flexibility to accommodate minor variations in the head size and shape of the wearer. Tightening and retention mechanisms may be provided to better secure the helmet to the wearer. Said tightening and retention mechanisms may comprise indicia such that the degree and orientation of fitting may be recorded and reproduced for separate and repeated instances of MEG analysis of the same wearer. This allows for provision of reusable helmets for a range of wearers.

The present invention further provides a method of manufacture or production of magnetoencephalography (MEG) apparatus comprising a helmet. A helmet is provided that is sized according to predetermined data. The helmet comprises at least one mount for retaining a magnetic field sensor in or on the helmet. The helmet may comprise a layer of material between the magnetic field sensor and the wearer when the magnetic field sensor is mounted to the helmet. Alternatively, the helmet may comprise an aperture defined by the mount such that the magnetic field sensor may be touching the anatomy of the user, or in physical proximity to the anatomy of the wearer with an air of vapour gap therebetween.

The helmet will be of known geometry, or the geometry of the helmet will be accurately measured using imaging techniques. Preferably, 3D imaging will be used. Medical imaging data defining the nature and location of anatomical features of the wearer is recorded. An image of the subject's head is provided. To accurately determine the location of sensors required by the MEG procedure, the anatomy of the subject's head is mapped to the geometry of the helmet so that the location and orientation of each of the magnetic field sensors is known with respect to the structure of the brain. The accuracy of the location may be determined according to the medical imaging method used. Each of the sensors is then applied at a predetermined position, determined during the mapping step. The distance of the sensors from an inside surface of the helmet, or from another datum point such as the sensor mount of patient skull, is determined and set according to pre-determined data, the purpose behind the MEG, clinical diagnosis or other relevant factor. The MEG process may be undertaken and the distance of the sensors from the relevant data adjusted according to the quality of the results, and/or according to the need for more or less detailed information in subsequent imaging and/or measuring iterations.

### BRIEF DESCRIPTION OF THE DRAWINGS

The invention will now be described by way of example and with reference to the accompanying figures, in which;
Figure 1 is a schematic exploded view of a mechanism for mounting a sensor to a helmet such that the sensor is movable towards or away from the interior of the helmet in accordance with the invention;
Figure 2 is a side elevation view of the mechanism of Figure 1;
Figure 3 (a) to 3(c) are plan views of each of the elements of the mechanism of Figures 1 and 2, and
Figure 4 is a photograph of a helmet for mounting the mechanism of Figures 1 to 3.

### DETAILED DESCRIPTION OF THE EMBODIMENTS

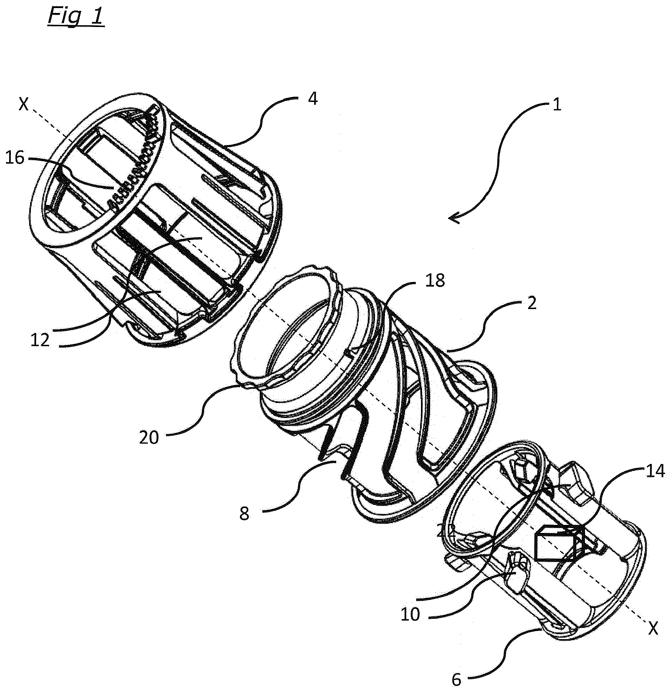

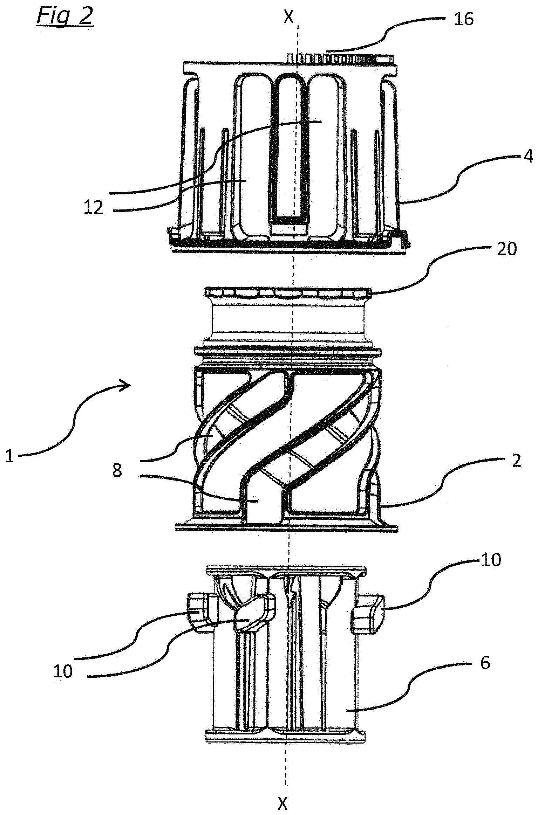

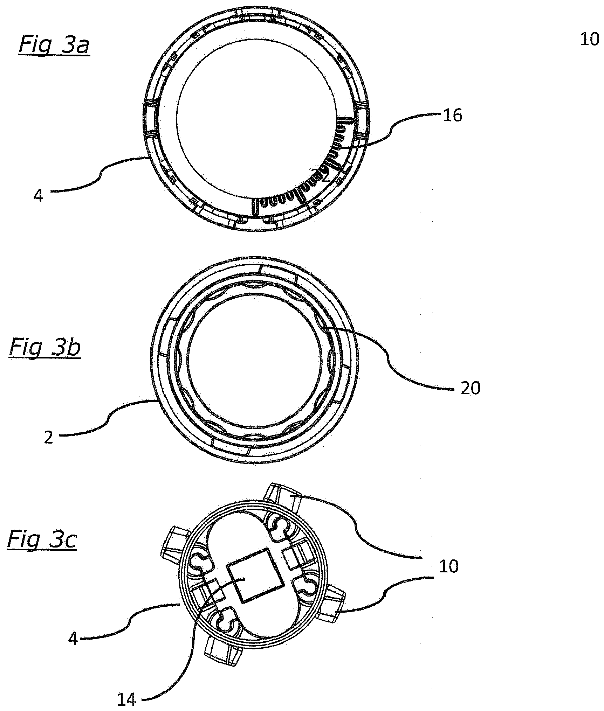

Figure 1 shows in exploded view a mechanism 1 for mounting a sensor to a helmet such that the sensor is movable towards or away from the interior of the helmet, to provide an MEG apparatus in accordance with the invention. Figures 2 and 3 are side elevation and plan views of the same mechanism 1. The mechanism 1 comprises three main elements, an inner collar 2, an outer collar 4 and an inner slider 6; all are generally cylindrical in shape, and are illustrated exploded along the common longitudinal or cylindrical axis XX about which they are assembled in use. In use, the three elements are nested with the outer collar 4 outermost and the slider 6 innermost, with the inner collar 2 sandwiched between them. The inner collar 2 is provided with a number of spiral cutouts which form pathways for lugs 10 on the exterior of the slider 6 to slidingly engage. Outer collar 4 is provided with longitudinal slots 12 which also slidingly engage with lugs 10 (which lugs are sufficiently long in the radial direction to simultaneously engage with both the cutouts and the slots, so that the lugs are free to slide relative to both, there being sufficient tolerance between the lugs and the cutouts and slots to permit this but to prevent and significant loose "play").

When the three elements 2, 4, 6 are assembled in their nested positions and the outer collar 4 is rotated relative to the inner collar 2 (or *vice versa*)*,* the slots 12 will urge the lugs 10 on the slider 6 to move in a circular direction about the longitudinal axis. Because the lugs 10 are held within the spiral cutouts 8, the lugs 10 will also follow their spiral path, and the inner slider 6 will both rotate and translate longitudinally along the axis of the mechanism 1, causing an MEG sensor (shown schematically at 14) held within the slider 6 to be moved along the common axis. The amount of axial movement will be a function of the angle of the spiral cutouts 8 to the axis and the amount of relative rotary movement between the inner and outer collars 2, 4. There are a number of radial markers 16 disposed around the edge of the outer collar 4, and when the mechanism 1 is assembled a marker 18 provided on the outer edge of the inner collar 2 is aligned with the radial markers 16 to give a visual indication of how far the slider 18 has moved along the axis; the spacing between the radial markers 16 is preferably chosen so as to correspond to a predetermined amount of longitudinal movement of the slider 6 (for example, the radial markers 16 may be disposed and the spiral angle of the cutouts 8 chosen so that the circumferential distance between adjacent radial markers equates to a longitudinal movement of the slider 6 of 0.1mm, or 0.5mm, or 1mm).

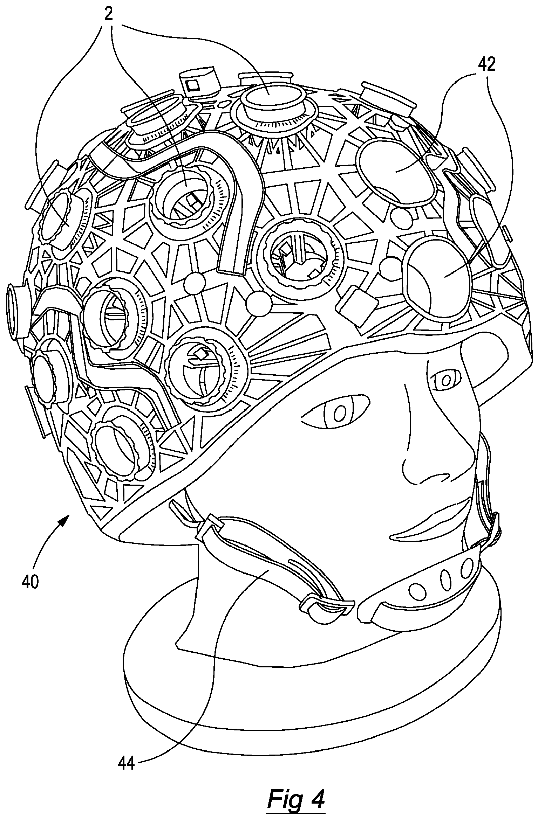

The inner collar 2 may be fixed in use and the outer collar 4 rotated by gripping the exterior of the outer collar 4, or the outer collar 4 could be fixed and the inner collar 2 rotated using the knurling 20 at the top of the inner collar 2. Figure 4 shows a helmet 40 which is provided with multiple openings 42, each of which is shaped and configured to receive and releasably hold a mechanism 1 as described above (in Figure 4, the openings 42 are adapted to receive the inner collar 2 of the mechanism, but they could equally be adapted to receive the outer collar 4 instead). Each mechanism 1 may have an outer collar 4 and a slider 6, so as to retain an MEG field sensor (not shown in Figure 4) which can be accurately moved inwardly or outwardly in relation to the helmet 40; the shape and configuration of the helmet 40 and its openings 42 are precisely manufactured so that the position and axial orientation of the openings 42 is known with a high degree of accuracy, so that the position and orientation of the MEG field sensor relative to the head of the subject inside the helmet 40 can be easily and accurately determined. The use of rotational movement to drive axial movement of the sensor is inherently resistant to reversal, which is advantageous because it means that the sensor will resist being pushed axially out of its desired position. The helmet 40 is formed in a lattice structure, which means the helmet is light to wear and also allows heat generated by the sensors to dissipate. In some embodiments, there is a chinstrap 44 which holds the helmet in a fixed position relative to the subject's head.

In a preferred embodiment, the helmet is a 3D-printed helmet 40 based on average adult head sizes. The helmet geometry may be defined based on standard size and shape data of certain human characteristics, including age, sex, and/or medical condition affecting the size and shape of the skull. The helmet is formed of a rigid material. Any suitable known material may be selected. The rigidity of the structure holds the sensors in a fixed position with respect to the wearer, and in a fixed orientation with respect to each other.

In a further embodiment, the helmet may be fabricated by casting, by a plastic injection moulding process, or similar such methods.

In order to accurately define the location of the sensor mounting mechanism 1 with respect to the underlying anatomy of the wearer, and corelate this information to specific locations on the helmet, data captured by imaging can be used to register the known shape of the helmet, and to determine the features of the wearer's anatomy. The location of specific aspects of anatomy, including location of bone and soft tissue, of the wearer can be mapped to an appropriate granularity. The location of action potentials and individual neurons may be determined according to the scanning technique used to record the relevant anatomical data.

Any suitable technique may be used to determine the shape and dimension of the helmet, including 3D scanning using cameras or other equipment, or simply design data taken from manufacturing logs and CAD models. Anatomical data may be captures using any available medical imaging process.

Once the data relating to the geometry of the helmet and the wearers anatomical features is determined, the position and orientation of the sensor array with respect to the head can be determined and recorded. The location of each sensor relative the underlying anatomical structure is recorded and used to determine the nature and cause of the magnetic activity in the brain.

As the OPMs can reach temperatures of around 40 degrees, in a preferred embodiment, the helmet is designed with an open 'lattice' structure to allow for air flow which carries the heat away from the head. The inventors have noted a high rate of success with the scanning of adults.

In an alternative embodiment, particularly where non-standard head size and shape is present in the user, the helmet may be formed of a more flexible material. The helmet may be adjusted to fit the user by means of known retention and adjustment mechanisms, some of which may include the means to record the adjustments required to fit the helmet to a specific wearer. The dimensions of the helmet may then be recorded while the helmet is fitted o the searer, and the underlying anatomy of the wearer mapped to the helmet accordingly.

The use of flexible material is possible because the mount for each magnetic field sensor is of a fixed orientation and variable axial position. Consequently, the proximity of each magnetic field sensor is unaffected by pressure of the head on the inside surface of the helmet, or by resilience in the helmet material or construction.

It will of course be understood that many variations may be made to the above-described embodiment without departing from the scope of the present invention. For example, there could be fewer or more cutouts, lugs and slots than are shown in the drawings (although we find that four provides sufficient accuracy of movement and positioning while not introducing too much friction to inhibit easy movement of the elements), and the spiral angle of the cutouts can be chosen to increase or decrease the amount of axial movement relative to the relative rotational movement of the elements

Where different variations or alternative arrangements are described above, it should be understood that embodiments of the invention may incorporate such variations and/or alternatives in any suitable combination.

## Claims

1. Magnetoencephalography (MEG) apparatus comprising a helmet shaped and configured to fit a range of human head sizes and/or shapes, the helmet comprising, a plurality of openings disposed in predetermined locations around the helmet, each opening being adapted to receive a magnetoencephalography field sensor in an arrangement such that the magnetoencephalography field sensor is moveable in a direction towards or away from a human head inside the helmet.

2. MEG apparatus according to claim 1, wherein the helmet further comprises a fixture for fixing the helmet securely in position on a human head,

3. MEG apparatus according to claim 1 or claim 2, wherein the magnetoencephalography field sensor is an Optically Pumped Magnetometer (OPM).

4. MEG apparatus according to any one of claims 1 to 3 comprising at least two magnetoencephalography field sensors, each received in an opening in the helmet, in which at least one of the magnetoencephalography field sensors and/or the opening in which it is received is/are provided with indicia showing the position of the magnetoencephalography field sensor relative to the opening in the direction towards or away from a human head inside the helmet.

5. MEG apparatus according to any preceding claim in which the or each magnetoencephalography field sensor is adapted to move in a spiral path relative to helmet to move in the direction towards or away from a human head inside the helmet.

6. MEG Apparatus according to any preceding claim in which the or each magnetoencephalography field sensor is mounted in a housing which is releasably mountable within the openings in the helmet, the sensor being movable with relation to the housing so as to provide the capability of movement of the magnetoencephalography field sensor in a direction towards or away from a human head inside the helmet when the housing is mounted to an opening.

7. MEG apparatus according to Claim 5 in which at least one of the magnetoencephalography field sensors and/or the housing(s) in which it is/they are mounted is/are provided with indicia showing the position of the magnetoencephalography field sensor relative to the housing in the direction towards or away from a human head inside the helmet

8. MEG apparatus according to any preceding claim in which the helmet is provided with apertures between the openings.

9. MEG apparatus according to Claim 7 in which the helmet has an open lattice structure surrounding the openings.

10. A kit of parts comprising a helmet according to any one of claims 1 to 9, at least one magnetoencephalography field sensor, and a mount comprising a housing which is releasably mountable within the openings in the helmet for retaining the at least one magnetoencephalography field sensor therein according to any one of claims 6 to 7.
